# EUROPEAN PATENT APPLICATION

(11) **EP 4 846 954 A1**
(43) Date of publication of application: **02.12.2026**
(21) Application number: 24920689.7
(22) Date of filing: 14.10.2024
(51) Int. Cl.: A61B 17/221, A61F 2/86, A61F 2/89, A61F 2/88, A61B 17/00, A61B 17/22, A61F 2/95

(54) **MECHANICAL THROMBECTOMY DEVICE**

(30) Priority: 26.01.2024 KR 20240012201; 27.05.2024 KR 20240068281
(71) Applicant: Nventric Corporation, Seoul 08376 (KR)
(72) Inventor: NGO, Don Quy, Los Angeles, California 90065 (US); MIN, Sungwoo, Fullerton, California 92835 (US); MIN, Jiyoung, Seoul 04739 (KR)
(74) Representative: Bringer IP
(86) International application number: PCT/KR2024/015453
(87) International publication number: WO 2025/159273

(57) **Abstract**

A mechanical thrombectomy device is provided. The mechanical thrombectomy device includes a support wire, a clot arrestor including an expandable frame and connected to the support wire, and a locking member configured to couple the support wire and the clot arrestor. The locking member may include a clasp having a first ring-shaped portion at a first end of a semi-cylindrical shaped portion and a second ring-shaped portion at a second end of the semi-cylindrical shaped portion, wherein a slit is formed in each of the first ring-shaped portion and the second ring-shaped portion.

## Description

### [TECHNICAL FIELD]

The present disclosure relates to a thrombectomy device and, more particularly but not limited thereto, to a mechanical thrombectomy device used for removing a thrombus from a blood vessel.

### [BACKGROUND]

Various methods exist for treating a thrombus formed at a particular blood vessel site of a body. Among them, a mechanical thrombectomy device is inserted into a blood vessel to break up or remove the thrombus within the blood vessel and is used to restore perfusion through an occluded blood vessel. Mechanical thrombectomy devices approved for such purposes include thrombus filters, thrombus aspiration devices, coil retrievers, and more recently, stent retriever devices.

A stent retriever device is primarily used to remove an acute thrombus from a stroke patient. A self-expanding stent formed of a wire mesh is deployed in a blood vessel to capture the thrombus, and the stent is then retrieved from the patient together with the thrombus by pulling a support wire of the stent retriever. In terms of usability and performance, such a stent retriever device is required to provide visibility during fluoroscopy, to capture, restrain, or retain a thrombus without damaging a blood vessel in a narrow and tortuous vascular structure, and to avoid creating dangerous situations during a procedure.

In a conventional stent retriever device, when the device is retrieved through a tortuous vascular structure, the strength of a connection portion between a clot arrestor and a support wire is weakened due to an increase in traction force and fatigue caused by repeated deformation, thereby causing a problem in that coupling between the clot arrestor and the support wire may be released during a procedure.

In addition, the conventional stent retriever device does not have a structure capable of sufficiently securing the strength of the connection portion between the support wire and the clot arrestor, and therefore cannot optimally transmit a pushing force and a traction force from the support wire to the clot arrestor.

### [SUMMARY]

In a conventional thrombectomy device, a connection structure between a support wire and a clot arrestor is not sufficiently strong, resulting in a problem in that coupling between the support wire and the clot arrestor may be released during a procedure. In addition, due to this problem, the traction force and pushing force of the support wire cannot be optimally transmitted to the clot arrestor.

An object of the present disclosure for solving the above-described problems is to provide a thrombectomy device having high coupling strength by reinforcing a coupling force between a support wire and a clot arrestor through the provision of a locking portion between the support wire and the clot arrestor.

Another object of the present disclosure for solving the above-described problems is to provide a thrombectomy device in which the strength of a coupling portion between a support wire and a clot arrestor is maintained sufficiently high even during repeated deformation, without increasing a radial profile of the coupling portion between the support wire and the clot arrestor.

However, the technical problems to be solved by the present disclosure are not limited thereto, and may be variously expanded without departing from the spirit and scope of the present disclosure.

A thrombectomy device according to one embodiment of the present disclosure for achieving the above-described objects is a mechanical thrombectomy device including: a support wire; a clot arrestor including an expandable frame and connected to the support wire; and a locking portion configured to couple the support wire and the clot arrestor, in which the locking portion may include a clasp having a first ring-shaped portion at a first end of a semi-cylindrical shaped portion and a second ring-shaped portion at a second end of the semi-cylindrical shaped portion, wherein a slit is formed in each of the first ring-shaped portion and the second ring-shaped portion.

According to one aspect, the clasp may at least partially surround a proximal portion of the clot arrestor and a distal portion of the support wire, and the support wire may be disposed in the slit of each of the first ring-shaped portion and the second ring-shaped portion.

According to one aspect, the slit may have opposing inclined surfaces that are in contact with a surface of the support wire.

According to one aspect, a minimum width of the slit may be formed to be smaller than a maximum cross-sectional length of the support wire.

According to one aspect, the proximal portion of the clot arrestor may be coupled so as to be engaged between the first ring-shaped portion and the second ring-shaped portion.

According to one aspect, the proximal portion of the clot arrestor may include a T-shaped tail portion fitted between the first ring-shaped portion and the second ring-shaped portion.

According to one aspect, a transverse length of the T-shaped tail portion perpendicular to a longitudinal direction of the proximal portion of the clot arrestor may be greater than inner diameters of the semi-cylindrical shaped portion, the first ring-shaped portion, and the second ring-shaped portion of the clasp, and may be smaller than or equal to outer diameters of the semi-cylindrical shaped portion, the first ring-shaped portion, and the second ring-shaped portion of the clasp.

According to one aspect, a distal end of the support wire may include a stepped portion having a maximum cross-sectional length greater than a maximum cross-sectional length of the support wire.

According to one aspect, the stepped portion may be tapered in a direction toward a proximal portion of the support wire.

According to one aspect, the support wire may be disposed in the slit while covering the proximal portion of the clot arrestor disposed between the first ring-shaped portion and the second ring-shaped portion.

According to one aspect, the locking portion may further include a coil wound around the support wire to support the locking portion.

According to one aspect, the clasp may be disposed such that the first end contacts the stepped portion of the support wire and the second end contacts the coil, so as to be fixed.

According to one aspect, the locking portion may further include soldering applied to at least a portion of the coil, the distal portion of the support wire, the proximal portion of the clot arrestor, and at least a portion of the clasp so as to prevent separation of the distal portion of the support wire, the proximal portion of the clot arrestor, the coil, and the clasp.

According to one aspect, the soldering applied to the distal portion of the support wire and the proximal portion of the clot arrestor may be wrapped by a biocompatible adhesive at the stepped portion of the support wire.

According to one aspect, the adhesive may be tapered in a direction toward the clot arrestor.

A thrombectomy device according to one embodiment of the present disclosure for achieving the above-described objects is a mechanical thrombectomy device that may include: a support wire; a clot arrestor including an expandable frame and connected to the support wire; and a locking portion configured to couple the support wire and the clot arrestor.

According to one aspect, the locking portion may include a stepped portion formed at a distal end of the support wire, a tail portion formed at a proximal frame end of the expandable frame so as to be coupled to the stepped portion, and a locking mechanism configured to secure the support wire on which the stepped portion is formed and the proximal frame end on which the tail portion is formed.

According to one aspect, the support wire and the stepped portion may be tapered and may be made of nitinol.

According to one aspect, the stepped portion may have a cylindrical shape with a diameter greater than a diameter of the support wire, and may be formed such that one side of the cylindrical shape has a flat cross-section, thereby being configured to closely contact at least a portion of the tail portion and the proximal frame end.

According to one aspect, the stepped portion may include a catching surface formed at a connection portion with the support wire, the catching surface being configured such that at least a portion of the tail portion is caught thereon.

According to one aspect, the tail portion may include at least one cut-out configured to engage the catching surface.

According to one aspect, the cut-out may include a dual fin formed to have a curvature identical to a curvature of the stepped portion.

According to one aspect, the locking mechanism may include a coil wound around the support wire and the proximal frame end while the tail portion is engaged with the catching surface of the stepped portion, and soldering at least partially applied onto the coil.

According to one aspect, the tail portion may include hook-shaped branches bent such that ends thereof are joined together.

According to one aspect, the distal end of the support wire including the stepped portion may be inserted into a gap between the branches, and curved ends of the branches may be configured to engage the catching surface of the stepped portion.

According to one aspect, the locking mechanism may include a coil wound around the support wire and the proximal frame end in a state where the tail portion is engaged with the catching surface of the stepped portion, and soldering at least partially applied onto the coil.

According to one aspect, the tail portion may include a plurality of cylindrical ring-shaped portions configured to receive and support the support wire.

According to one aspect, a most distal ring-shaped portion among the ring-shaped portions may be configured to engage the catching surface of the stepped portion.

According to one aspect, the ring-shaped portion may include an opening, and at least some of a plurality of openings respectively provided in the plurality of cylindrical ring-shaped portions may be arranged in different orientations.

According to one aspect, the locking mechanism may include at least one of compressing the ring-shaped portion such that the support wire is firmly supported by the ring-shaped portion in a state where the support wire is received within the ring-shaped portion such that the ring-shaped portion engages the catching surface of the stepped portion, and soldering the ring-shaped portion at least partially.

According to one aspect, the locking portion may include a stepped portion formed at a distal end of the support wire and having a plurality of first through-holes, a tail portion formed at the proximal frame end of the expandable frame and having a plurality of second through-holes respectively corresponding to the plurality of first through-holes, and a locking mechanism configured to couple the support wire on which the stepped portion is formed and the proximal frame end on which the tail portion is formed.

According to one aspect, the support wire and the stepped portion may be tapered and may be made of nitinol.

According to one aspect, the stepped portion may have a cylindrical shape with a diameter greater than a diameter of the support wire, opposite sides of the cylindrical shape being formed to have flat cross-sections, and the plurality of first through-holes may be formed in the flat cross-sections.

According to one aspect, the tail portion may be arranged such that the plurality of second through-holes of the tail portion overlap the plurality of first through-holes of the stepped portion.

According to one aspect, the locking mechanism may include a coil wound around the stepped portion and the tail portion after the overlapping plurality of first through-holes and second through-holes of the stepped portion and the tail portion are stitched in a crossing manner.

According to one aspect, the locking mechanism may further include soldering at least partially applied onto the coil.

According to one aspect, an edge of the flat cross-section of the stepped portion may be ground or tapered.

The disclosed technology may provide the following effects. However, this does not mean that a particular embodiment must include all of the following effects or only the following effects. Accordingly, the scope of the disclosed technology should not be construed as being limited thereby.

According to the mechanical thrombectomy device according to one embodiment of the present disclosure described above, by providing the locking portion that couples the support wire and the clot arrestor, the coupling strength between the support wire and the clot arrestor is excellent despite an increase in fatigue caused by repeated deformation of the thrombectomy device. Accordingly, a safe thrombectomy device can be provided in which the risk of the clot arrestor becoming separated from the support wire during a procedure is reduced.

In addition, the mechanical thrombectomy device according to one embodiment of the present disclosure described above may provide the high-strength locking mechanism having flexibility without increasing the radial profile of a connection portion between the support wire and the clot arrestor. Accordingly, the mechanical thrombectomy device that maintains sufficient coupling strength within a patient's complex vascular structure while also maintaining flexibility can be provided.

### [BRIEF DESCRIPTION OF THE DRAWINGS]

FIG. 1 is a plan view of a mechanical thrombectomy device according to one embodiment of the present disclosure.
FIG. 2 is an enlarged perspective view of a locking portion according to one embodiment of the present disclosure.
FIG. 3 is a view illustrating a support wire from various angles according to one embodiment of the present disclosure.
FIG. 4 is a view illustrating a tail portion from various angles according to one embodiment of the present disclosure.
FIG. 5 is a view for explaining a locking mechanism according to one embodiment of the present disclosure.
FIG. 6 is a view illustrating a locking portion from various angles according to one embodiment of the present disclosure.
FIG. 7 is a view illustrating a tail portion from various angles according to one embodiment of the present disclosure.
FIG. 8 is a view for explaining a locking mechanism according to one embodiment of the present disclosure.
FIG. 9 is an exploded view of a locking portion according to one embodiment of the present disclosure.
FIG. 10 is a view illustrating a tail portion from various angles according to one embodiment of the present disclosure.
FIG. 11 is a view for explaining a locking mechanism according to one embodiment of the present disclosure.
FIG. 12 is an enlarged view of a locking portion according to one embodiment of the present disclosure.
FIG. 13 is a view illustrating a stepped portion from various angles according to one embodiment of the present disclosure.
FIG. 14 is a view illustrating a tail portion from various angles according to one embodiment of the present disclosure.
FIG. 15 is a view for explaining a locking mechanism according to one embodiment of the present disclosure.
FIG. 16 is an enlarged perspective view of a locking portion according to one embodiment of the present disclosure.
FIG. 17 is a view illustrating a clasp from various angles according to one embodiment of the present disclosure.
FIG. 18 is a cross-sectional view illustrating a state in which a clasp and a support wire are coupled according to one embodiment of the present disclosure.
FIG. 19 is a view for explaining a proximal portion of a clot arrestor according to one embodiment of the present disclosure.
FIG. 20 is a coupling view of a proximal portion of a clot arrestor and a clasp according to one embodiment of the present disclosure.
FIG. 21 is a coupling view of a proximal portion of a clot arrestor, a clasp, and a support wire according to one embodiment of the present disclosure.
FIG. 22 is a plan view of a locking portion according to one embodiment of the present disclosure.
FIG. 23 is a side view of a locking portion according to one embodiment of the present disclosure.

### [DETAILED DESCRIPTION]

The present disclosure may be modified in various ways and may have various embodiments, and specific embodiments will be illustrated in the drawings and described in detail.

However, this is not intended to limit the present disclosure to the specific embodiments, and it should be understood that the present disclosure includes all modifications, equivalents, and substitutes falling within the spirit and technical scope of the present disclosure.

Terms such as first and second may be used to describe various elements, but the elements should not be limited by these terms. These terms are used only for the purpose of distinguishing one element from another element. For example, without departing from the scope of the present disclosure, a first component may be referred to as a second component, and similarly, a second component may be referred to as a first component. The term "and/or" includes any combination of a plurality of related listed items or any one of the plurality of related listed items.

When an element is referred to as being "connected" or "coupled" to another element, it should be understood that it may be directly connected or coupled to the other element, or intervening elements may be present therebetween. In contrast, when an element is referred to as being "directly connected" or "directly coupled" to another element, it should be understood that no intervening element is present therebetween.

The terminology used herein is for the purpose of describing particular embodiments only and is not intended to limit the present disclosure. Singular forms include plural forms unless the context clearly indicates otherwise. As used herein, the terms "include" and "have" are intended to specify the presence of stated features, numbers, steps, operations, elements, components, parts, or combinations thereof, and should not be construed as precluding the presence or addition of one or more other features, numbers, steps, operations, elements, components, parts, or combinations thereof.

Unless otherwise defined, all terms used herein, including technical and scientific terms, have the same meanings as commonly understood by a person having ordinary skill in the art to which the present disclosure pertains. Terms defined in commonly used dictionaries should be interpreted as having meanings consistent with their meanings in the context of the relevant art and should not be interpreted in an idealized or overly formal sense unless expressly defined herein.

Hereinafter, preferred embodiments of the present disclosure will be described in greater detail with reference to the accompanying drawings. In the following description of the present disclosure, the same reference numerals are used for the same elements throughout the drawings, and redundant descriptions thereof will be omitted for clarity.

As discussed above, in a conventional stent retriever, when the device moves through a patient's complex vascular structure, accumulated fatigue due to repeated deformation may cause coupling at a connection portion between a clot arrestor and a support wire to be released, thereby creating a dangerous situation during a procedure. In addition, the conventional stent retriever device does not provide sufficient coupling strength at the connection portion between the support wire and the clot arrestor, and therefore cannot optimally transmit a traction force and a pushing force of the support wire to the clot arrestor during a procedure.

The mechanical thrombectomy device according to one embodiment of the present disclosure is intended to solve the above-described problems and may provide a mechanical thrombectomy device capable of reinforcing a coupling force between a clot arrestor and a support wire by providing a locking portion that couples the support wire and the clot arrestor, while optimally transmitting a traction force and a pushing force of the support wire to the clot arrestor without increasing a radial profile of a connection portion between the support wire and the clot arrestor.

Hereinafter, a mechanical thrombectomy device according to one embodiment will be described in more detail with reference to the drawings.

Hereinafter, in the present disclosure, a "clot arrestor" may refer, for example, to a portion of a general stent retriever including a stent. However, the clot arrestor is not limited to a conventional stent retriever and should be understood as including all components required to restore perfusion by removing coagulated blood from a blood vessel.

Further, hereinafter, in the present disclosure, an "expandable frame" refers to a frame that is initially maintained in a compressed state and may be expanded after being moved to an appropriate location, and it should be understood that any form known in the art may be applied thereto.

In addition, the use of relative terms throughout the Description of the Invention of the present specification may indicate relative positions or directions. For example, "distal" may indicate a first direction along a longitudinal axis of a support wire or a clot arrestor. Likewise, "proximal" may indicate a second direction opposite to the first direction. However, these terms are provided to establish relative references and are not intended to limit the use or orientation of the mechanical thrombectomy device to specific configurations described in the various embodiments below.

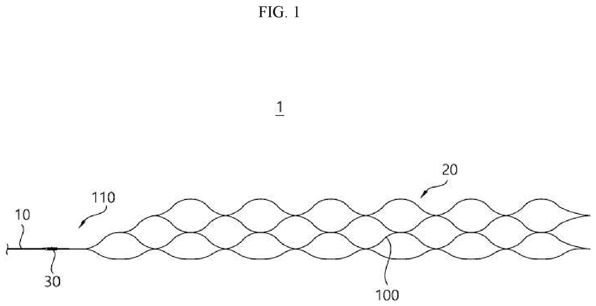

FIG. 1 is a plan view of a mechanical thrombectomy device 1 according to one embodiment of the present disclosure. As illustrated in FIG. 1, the mechanical thrombectomy device 1 according to one embodiment may include, for example, a support wire 10, a clot arrestor 20, and a locking portion 30.

The mechanical thrombectomy device 1 is, for example, an endovascular tool that may be used to treat acute ischemic stroke. The mechanical thrombectomy device 1 includes a proximal control region that is used by an operator to advance, retrieve, and rotate a distal operating region of the device. More specifically, the mechanical thrombectomy device 1 includes a support wire 10 that may be pushed by the operator to advance the distal operating region, pulled by the operator to retrieve the distal operating region, or rotated by the operator to rotate the distal operating region. In one embodiment, the support wire 10 may be a flexible elongated wire formed of a resilient material, such as stainless steel or super elastic nickel titanium alloy, for example, nitinol.

The mechanical thrombectomy device 1 may include the clot arrestor 20 that may be advanced through a microcatheter and deployed from the microcatheter to a target tissue. When deployed within the target tissue, the clot arrestor 20 may capture, entrap, restrain, or mechanically integrate with a thrombus. The restrained thrombus may then be retrieved from a patient by pulling the support wire 10 to retrieve the clot arrestor 20 and the thrombus from a vascular structure.

The clot arrestor 20 may be sized and shaped to provide respective degrees of thrombus restraint, thrombus capture, flexibility, or any other performance characteristic. The clot arrestor 20 may be connected to a distal end of the support wire 10 and may include an expandable frame 100. The expandable frame 100 may have a generally elongated cylindrical frame structure capable of capturing and restraining a thrombus within a cylindrical interior thereof, and may have flexibility, shape memory, and contraction properties. According to one aspect, the expandable frame 100 may be manufactured from a shape memory alloy material including a nickel-titanium alloy, although the present disclosure is not limited thereto. The size and shape of each cell of the expandable frame 100 may be determined according to the performance characteristics of the thrombectomy device described above. The clot arrestor 20 may be formed by laser-cutting a cylindrical tube into a three-dimensional expandable structure, or by machining, chemical machining, electro-machining, electrical discharge machining, or various methods known in the art, and the expandable frame 100 of the clot arrestor 20 may have, for example, a substantially circular cross-sectional profile.

Although not illustrated in the drawings, a plurality of radiopaque markers may be mounted on the expandable frame 100. The radiopaque markers may enhance the visibility of the device and provide an indication enabling an operator to determine a position of a thrombus relative to the clot arrestor. The radiopaque markers may include radiopaque markers mounted by various methods known in the art, including radiopaque bands, wire coils, plating, welding, and coating. Alternatively, a radiopaque material may be plated onto the expandable frame 100. Alternatively, the radiopaque markers may be formed as bands crimped onto the expandable frame 100. In one aspect, the radiopaque markers may include coils formed of radiopaque wire and may be wound or wrapped around the expandable frame 100. In one aspect, the radiopaque markers may have an atraumatic surface that does not damage a blood vessel wall during use.

The locking portion 30 is configured to couple the support wire 10 and the clot arrestor 20. The locking portion 30 may directly couple a proximal frame end 110 of the expandable frame 100 of the clot arrestor 20 and a distal end of the support wire 10 by various locking mechanisms. The locking portion 30 should be capable of providing sufficient flexibility and coupling strength to allow the mechanical thrombectomy device 1 to be naturally delivered through a tortuous vascular structure during use in a patient's body.

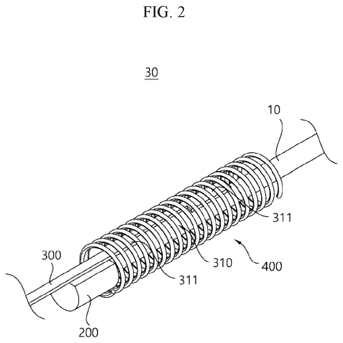

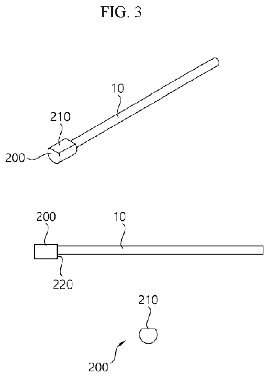

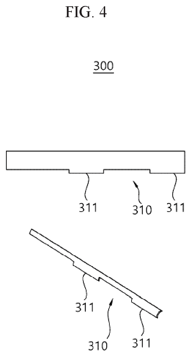

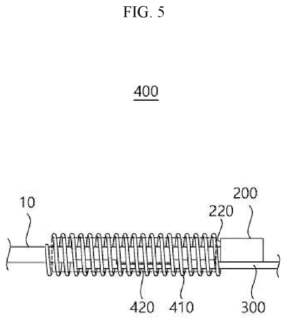

Hereinafter, the locking portion 30 according to one embodiment of the present disclosure will be described in detail with reference to FIGS. 2 to 5. FIG. 2 is an enlarged perspective view of the locking portion 30 according to one embodiment of the present disclosure, FIG. 3 is a view illustrating the support wire 10 from various angles according to one embodiment of the present disclosure, FIG. 4 is a view illustrating a tail portion 300 from various angles according to one embodiment of the present disclosure, and FIG. 5 is a view for explaining a locking mechanism 400 according to one embodiment of the present disclosure.

In the embodiment of FIG. 2, the locking portion 30 may include a stepped portion 200 formed at the distal end of the support wire 10, and may include the tail portion 300 formed at the proximal frame end 110 of the expandable frame 100 so as to be coupled to the stepped portion 200. The locking portion 30 may also include the locking mechanism 400 that fixes the support wire 10 on which the stepped portion 200 is formed and the proximal frame end 110 on which the tail portion 300 is formed.

The support wire 10 and the stepped portion 200 may be tapered. First, the support wire 10 may be a wire that is tapered continuously or stepwise from a proximal portion toward the distal end. For example, the distal portion of the support wire 10 connected to the stepped portion 200 may have a smaller diameter than the proximal portion of the support wire 10, and accordingly, the support wire 10 may have a stiffness profile decreasing in a distal direction. In addition, the stepped portion 200 formed at the distal end of the support wire 10 may have a diameter greater than a diameter of the support wire 10, but may also be tapered continuously or stepwise from the proximal portion toward the distal end. Accordingly, even after the support wire 10 and the tail portion 300 are fixed by the locking mechanism 400, a radial profile may be maintained compact. As described above with reference to FIG. 1, in one embodiment, the support wire 10 and the stepped portion 200 may be manufactured from a resilient material such as stainless steel or a super elastic nickel titanium alloy having flexibility, shape memory, and contraction properties, for example, nitinol.

More specifically, referring to FIG. 3, the stepped portion 200 may have a cylindrical shape having a diameter greater than the diameter of the support wire 10. By forming the diameter of the stepped portion 200 to be greater than the diameter of the support wire 10, the tail portion 300 and the locking mechanism 400 may be caught on a cross-sectional area of a step generated due to the diameter difference, thereby more securely coupling the support wire 10 and the tail portion 300. The stepped portion 200 may be formed such that one side of the cylindrical shape has a flat cross-section 210. Due to the flat cross-section 210, when the locking portion 30 is coupled, at least a portion of the tail portion 300 and the proximal frame end 110 of the clot arrestor 20 may be brought into close contact with the stepped portion 200, and the overall radial profile of the locking portion 30 may also be maintained compact. Referring to the side view of the support wire 10 illustrated in FIG. 3, the stepped portion 200 may include a catching surface 220 at a junction with the support wire 10. The catching surface 220 indicates an end surface of the stepped portion 200 excluding a diameter portion of the support wire 10 at an end connected to the support wire 10, and may be formed such that at least a portion of the tail portion 300 is caught on the catching surface 220 when the locking portion 30 is coupled.

Referring to FIGS. 4 and 5, the tail portion 300 may include at least one cut-out 310 caught on the catching surface 220. The cut-out 310 may be formed by at least partially cutting out the tail portion 300 along a longitudinal length of the tail portion 300. In one aspect, the cut-outs 310 may be symmetrically formed on both side surfaces of the tail portion 300, so that at least two cut-outs 310 may be formed. Due to the cut-out portion of the cut-out 310, dual fins may be formed at both ends of the cut-out 310 in the longitudinal direction of the tail portion 300. The dual fins 311 may be formed to have the same curvature as that of the stepped portion 200 so as to be brought into close contact with the catching surface 220 of the stepped portion 200. In one aspect, the dual fins 311 may also be symmetrically formed on both side surfaces of the tail portion 300, so that at least two dual fins 311 may be formed, and may have a larger cross-sectional area than the cut-out 310.

The locking mechanism 400 according to one embodiment of the present disclosure will be described with reference to FIG. 5. The stepped portion 200 and the tail portion 300 formed as described with reference to FIGS. 2 to 4 may be coupled to each other so as to be in close contact with each other in the state illustrated in FIG. 5. More specifically, the locking mechanism 400 may include a coil 410 firmly wound around the support wire 10 and the proximal frame end 110 of the tail portion 300 in a state in which the dual fins 311 of the tail portion 300 are caught on the catching surface 220 of the stepped portion 200. Although not illustrated in detail in FIG. 5, in one aspect, when the coil 410 is wound around the support wire 10 and the tail portion 300, the coil 410 may be firmly wound with a smaller diameter at a portion of the cut-out 310 of the tail portion 300 than at other portions due to the smaller cross-sectional area of the cut-out 310. Accordingly, the tail portion 300 may be fixed without sliding toward the proximal portion of the support wire 10 in a state of being in close contact with the support wire 10. More specifically, the dual fins 311 of the tail portion 300 having a larger cross-sectional area are caught on the coil 410 having a smaller diameter at the cut-out 310, thereby preventing the tail portion 300 from moving from the stepped portion 200 toward the proximal portion of the support wire 10. In one aspect, one end of the coil 410 may be wound so as to be caught on the catching surface 220 of the stepped portion 200, thereby also preventing separation of the coil 410. In one aspect, the coil 410 may be formed of stainless steel, platinum-iridium, or another radiopaque metal or material visible during fluoroscopy, and may act as a radiopaque marker.

In one aspect, the locking mechanism 400 may also include soldering 420 at least partially applied onto the coil 410. As described above, in a state in which the coil 410 is wound around the proximal frame end 110 of the tail portion 300 and the support wire 10, the soldering 420 may be applied to at least a portion of the coil 410. The soldering 420 may generally be formed of an alloy material including lead, tin, or silver, and in one aspect may be formed of a material including a radiopaque material to act as a radiopaque marker. The soldering 420 may flow into gaps of the coil 410 wound around the support wire 10 and the proximal frame end 110 of the tail portion 300, thereby further strengthening coupling among the support wire 10, the tail portion 300, and the coil 410. A diameter and a length of the soldering 420 may be differently determined according to coupling strength and flexibility required for the mechanical thrombectomy device 1.

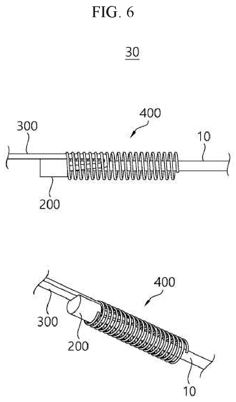

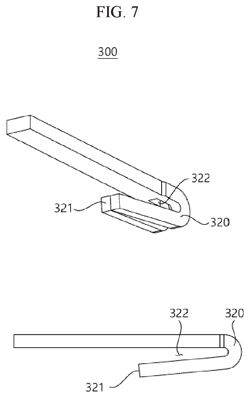

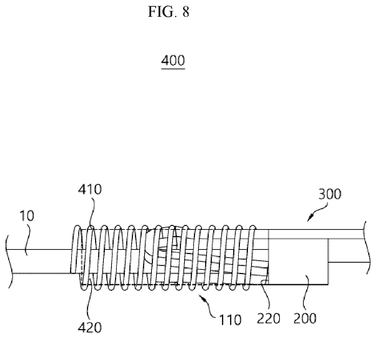

FIG. 6 is a view illustrating the locking portion 30 from various angles according to one embodiment of the present disclosure, FIG. 7 is a view illustrating the tail portion 300 from various angles according to one embodiment of the present disclosure, and FIG. 8 is a view for explaining the locking mechanism 400 according to one embodiment of the present disclosure. Hereinafter, another locking structure according to one embodiment of the present disclosure will be described with reference to FIGS. 6 to 8. Detailed configurations of the support wire 10 and the stepped portion 200 may have the same characteristics as described above, and thus redundant descriptions thereof will be omitted.

Referring to FIG. 6, in one embodiment of the present disclosure, similarly to the embodiment illustrated in FIGS. 2 to 5, the locking portion 30 may include the stepped portion 200 formed at the distal end of the support wire 10, and may include the tail portion 300 formed at the proximal frame end 110 of the expandable frame 100 so as to be coupled to the stepped portion 200. The locking portion 30 may also include the locking mechanism 400 that fixes the support wire 10 on which the stepped portion 200 is formed and the proximal frame end 110 on which the tail portion 300 is formed. However, according to one aspect, the tail portion 300 may have a hook-shaped end bent to merge into one.

Referring to FIG. 7, the tail portion 300 may include hook-shaped branches 320 having ends 321 bent to merge into one. As illustrated in FIG. 7, the tail portion 300 may include a gap 322 between the branches 320 into which the support wire 10 can be inserted. In addition, the tail portion 300 may further include a gap 322 into which the support wire 10 may be inserted between the ends 321 bent to merge into one and the branches 320 extending in a linear form.

Accordingly, as illustrated in FIG. 8, the support wire 10 may be inserted into the gap 322 between the branches 320. In this case, the support wire 10 may be inserted in a direction in which the bent ends 321 of the branches 320 and the catching surface 220 of the stepped portion 200 meet each other. That is, the end 321 of the tail portion 300 contacts and is caught on the catching surface 220 of the stepped portion 200, thereby primarily forming coupling between the support wire 10 and the tail portion 300. According to one aspect, after the support wire 10 is inserted into the gap 322, the branches 320 may be pressed so that the support wire 10 is firmly fixed in the gap 322. In this case, not only can the radial profile of the coupled support wire 10 and tail portion 300 be made compact, but coupling between the tail portion 300 and the support wire 10 may also be further strengthened. In addition, in one aspect, the branches 320 may be tapered continuously or stepwise toward the ends 321 so as to be thinner than other portions of the tail portion 300, and accordingly, the radial profile of the locking portion 30 may also be made compact.

In the embodiment illustrated in FIG. 8, the locking mechanism 400 may include the coil 410 wound around the support wire 10 and the proximal frame end 110 in a state in which the tail portion 300 is caught on the catching surface 220 of the stepped portion 200, and the soldering 420 at least partially applied onto the coil 410. Although not illustrated in detail in FIG. 8, when the coil 410 is wound around the support wire 10 and the tail portion 300, the coil 410 may be firmly wound to have a step with a smaller diameter on the support wire 10 at a portion where the branches 320 do not extend, compared to a portion where the branches 320 extend. Accordingly, due to the smaller diameter of the coil 410, the tail portion 300 may be fixed without sliding toward the proximal portion of the support wire 10 in a state of being in contact with the support wire 10. That is, the branches 320 of the tail portion 300 are caught on the coil 410 having the smaller diameter, thereby preventing the tail portion 300 from moving from the stepped portion 200 toward the proximal portion of the support wire 10, and thus coupling between the support wire 10 and the tail portion 300 can be further strengthened. The coupling of the branches 320 on the support wire 10 may be reinforced several times by the catching surface 220 of the stepped portion 200, the smaller-diameter portion of the coil 410, and pressing of the branches 320 as described above. In one aspect, the coil 410 may be formed of stainless steel, platinum-iridium, or another radiopaque metal or material visible during fluoroscopy, and may act as a radiopaque marker.

In one aspect, the locking mechanism 400 may also include the soldering 420 at least partially applied onto the coil 410. As described above, in a state in which the coil 410 is wound around the support wire 10 and the proximal frame end 110, the soldering 420 may be applied to at least a portion of the coil 410. The soldering 420 may generally be formed of an alloy material including lead, tin, or silver, and in one aspect may be formed of a material including a radiopaque material to act as a radiopaque marker. The soldering 420 may flow into gaps of the coil 410 wound around the support wire 10 and the proximal frame end 110 of the tail portion 300, thereby further strengthening coupling among the support wire 10, the tail portion 300, and the coil 410. A diameter and a length of the soldering 420 may be differently determined according to coupling strength and flexibility required for the mechanical thrombectomy device 1.

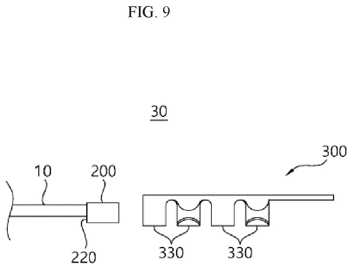

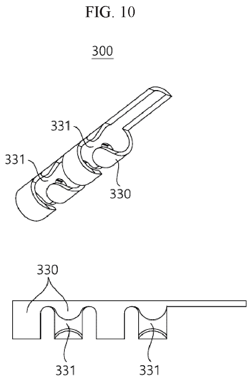

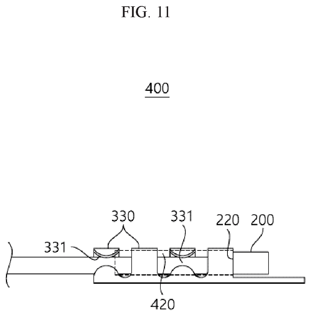

FIG. 9 is an exploded view of the locking portion 30 according to one embodiment of the present disclosure, FIG. 10 is a view illustrating the tail portion 300 from various angles according to one embodiment of the present disclosure, and FIG. 11 is a view for explaining the locking mechanism 400 according to one embodiment of the present disclosure. Hereinafter, still another locking structure according to one embodiment of the present disclosure will be described with reference to FIGS. 9 to 11. Detailed configurations of the support wire 10 and the stepped portion 200 may have the same characteristics as described above, and thus redundant descriptions thereof will be omitted.

In one embodiment of the present disclosure illustrated in FIGS. 9 to 11, similarly to the above-described embodiments, the locking portion 30 may include the stepped portion 200 formed at the distal end of the support wire 10, and may include the tail portion 300 formed at the proximal frame end 110 of the expandable frame 100 so as to be coupled to the stepped portion 200. The locking portion 30 may also include the locking mechanism 400 that fixes the support wire 10 on which the stepped portion 200 is formed and the proximal frame end 110 on which the tail portion 300 is formed. However, according to one aspect, the tail portion 300 may include a plurality of cylindrical ring-shaped portions 330 that receive and support the support wire 10.

As illustrated in FIGS. 9 to 11, each ring-shaped portion 330 has a ring shape sized to receive the support wire 10, and the distalmost ring-shaped portion 330 among the ring-shaped portions 330 may be formed to be caught on the catching surface 220 of the stepped portion 200, as illustrated in FIG. 11. In addition, each ring-shaped portion 330 may include an opening 331, and at least some of a plurality of openings 331 provided in the plurality of cylindrical ring-shaped portions 330 may be arranged in different orientations. For example, although the openings 331 are illustrated in FIGS. 9 to 11 as having the same orientation in an alternating manner, all of the openings 331 may be arranged in different directions. Due to the different orientations of the openings 331, when the device is used in various vascular structures, the support wire 10 may receive an overall uniform support force without being separated through the openings 331.

In the embodiment illustrated in FIG. 11, the locking mechanism 400 may include at least one of compressing the ring-shaped portions 330 so that the support wire 10 is firmly supported by the ring-shaped portions 330 in a state in which the support wire 10 is received in the ring-shaped portions 330 such that the ring-shaped portion 330 is caught on the catching surface 220 of the stepped portion 200, and applying soldering 420 to at least a portion of the ring-shaped portions 330. In one aspect, the ring-shaped portions 330 may be compressed on the support wire 10 to a diameter equal to the diameter of the stepped portion 200 so that the ring-shaped portions 330 firmly clamp the support wire 10. In addition, coupling between the support wire 10 and the tail portion 300 may be further strengthened by at least partially applying the soldering 420 onto the compressed ring-shaped portions 330. The soldering 420 may generally be formed of an alloy material including lead, tin, or silver, and in one aspect may be formed of a material including a radiopaque material to act as a radiopaque marker. The soldering 420 may flow between the ring-shaped portions 330 and into the openings 331, thereby further strengthening coupling between the support wire 10 and the ring-shaped portions 330 of the tail portion 300. A diameter and a length of the soldering 420 may be differently determined according to coupling strength and flexibility required for the mechanical thrombectomy device 1.

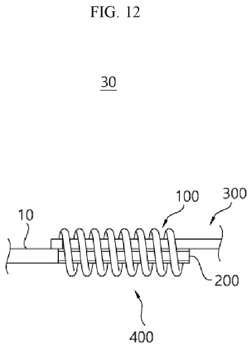

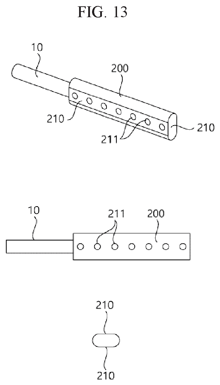

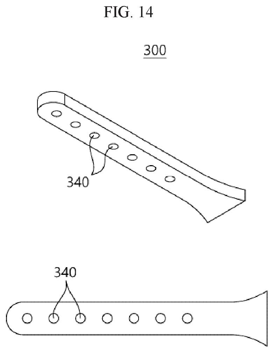

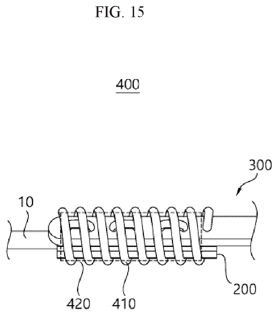

FIG. 12 is an enlarged view of the locking portion 30 according to one embodiment of the present disclosure, FIG. 13 is a view illustrating the stepped portion 200 from various angles according to one embodiment of the present disclosure, FIG. 14 is a view illustrating the tail portion 300 from various angles according to one embodiment of the present disclosure, and FIG. 15 is a view for explaining the locking mechanism 400 according to one embodiment of the present disclosure. Hereinafter, another locking portion according to one embodiment of the present disclosure will be described with reference to FIGS. 12 to 15.

In one embodiment of the present disclosure illustrated in FIGS. 12 to 15, similarly to the above-described embodiments, the locking portion 30 may include the stepped portion 200 formed at the distal end of the support wire 10, and may include the tail portion 300 formed at the proximal frame end 110 of the expandable frame 100 so as to be coupled to the stepped portion 200. The locking portion 30 may also include the locking mechanism 400 that couples the support wire 10 on which the stepped portion 200 is formed and the proximal frame end 110 on which the tail portion 300 is formed. However, according to one aspect, the locking portion 30 may include the stepped portion 200 formed at the distal end of the support wire 10 to have a plurality of first through-holes 211, and the tail portion 300 formed at the proximal frame end 110 of the expandable frame 100 to have a plurality of second through-holes 340 respectively corresponding to the plurality of first through-holes 211. As described above, in one embodiment, the support wire 10 and the stepped portion 200 may be manufactured from a resilient material such as stainless steel or a super elastic nickel titanium alloy having flexibility, shape memory, and contraction properties, for example, nitinol.

Referring to FIG. 13, the stepped portion 200 may be formed in a cylindrical shape having a diameter greater than the diameter of the support wire 10, and may be formed such that both sides of the cylindrical shape have flat cross-sections 210. In one aspect, the flat cross-sections 210 on both sides may be spaced apart by a distance equal to the diameter of the support wire 10, so that when the support wire 10 is viewed from the side as illustrated in FIG. 12, the stepped portion 200 and the support wire 10 may have the same height. The plurality of first through-holes 211 may be formed in the flat cross-section 210, and by forming the stepped portion 200 to have a diameter greater than that of the support wire 10, the strength of the stepped portion 200 may be maintained despite the formation of the plurality of first through-holes 211. In addition, corners of the flat cross-section 210 of the stepped portion 200 may be ground or tapered, thereby obtaining a more compact radial profile and excellent coupling characteristics when the locking mechanism is formed later.

Referring to FIG. 14, the tail portion 300 also has a flat cross-section so as to abut and contact the stepped portion 200, and may have substantially the same length and width as the ground or tapered flat cross-section 210 of the stepped portion 200. In one aspect, the tail portion 300 may be gradually or stepwise ground or tapered toward the proximal portion. The plurality of second through-holes 340 may be formed in the flat cross-section of the tail portion 300 so as to respectively correspond to the plurality of first through-holes 211 of the stepped portion 200. The first through-holes 211 and the second through-holes 340 may be arranged to overlap each other, and the first through-holes 211 and the second through-holes 340 may have a size capable of receiving the locking mechanism, for example, a diameter through which a coil can pass.

Referring to FIG. 15, the locking mechanism 400 may include the coil 410 that couples, for example, by suturing, the stepped portion 200 and the tail portion 300. First, in a state in which the flat cross-sections of the stepped portion 200 and the tail portion 300 abut each other such that the corresponding plurality of through-holes 211 and 340 are aligned with each other, the coil 410 may be formed to cross and suture together the plurality of overlapping first through-holes 211 and second through-holes 340 of the stepped portion 200 and the tail portion 300. In one aspect, such suturing may be formed in a linear form that sequentially passes through the through-holes 211 and 340 one by one above and below the flat cross-section from one end to the other end of each of the through-holes 211 and 340. However, the form of the suturing may vary depending on a desired stiffness profile or strength between the support wire 10 and the tail portion 300.

In addition, after all of the plurality of through-holes 211 and 340 of the stepped portion 200 and the tail portion 300 are sutured, the coil 410 may be entirely wound around the stepped portion 200 and the tail portion 300. The coil 410 may be a coil that is integrally extended from a coil suturing the first through-holes 211 and the second through-holes 340 and is wound. In this case, since the coil 410 is organically entangled on the support wire 10 and the tail portion 300, the coupling strength is high, and even if a portion of the coil 410 is broken, the mechanical thrombectomy device 1 may not be easily damaged. Alternatively, the plurality of through-holes 211 and 340 may first be sutured with a separate wire, and after the through-holes 211 and 340 are sutured, a separate coil may be wound around the stepped portion 200 and the tail portion 300. In one aspect, the coil 410 may be formed of stainless steel, platinum-iridium, or another radiopaque metal or material visible during fluoroscopy, and may act as a radiopaque marker.

In one aspect, the locking mechanism 400 may further include the soldering 420 at least partially applied onto the coil 410. As illustrated in FIG. 15, in a state in which the coil 410 is wound around the stepped portion 200 and the tail portion 300, the soldering 420 may be applied to at least a portion of the coil 410. The soldering 420 may generally be formed of an alloy material including lead, tin, or silver, and in one aspect may be formed of a material including a radiopaque material to act as a radiopaque marker. The soldering 420 may flow into gaps of the coil 410 wound around the support wire 10 and the proximal frame end 110 of the tail portion 300, thereby further strengthening coupling among the stepped portion 200, the tail portion 300, and the coil 410. A diameter and a length of the soldering 420 may be differently determined according to coupling strength and flexibility required for the mechanical thrombectomy device 1.

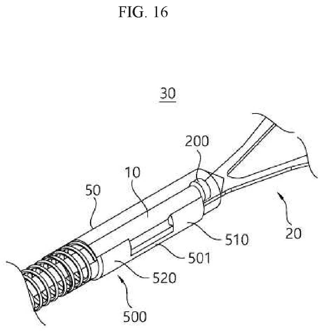

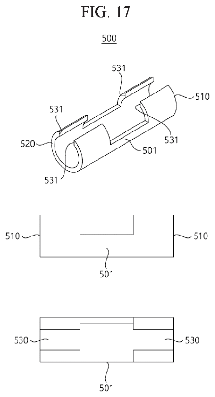

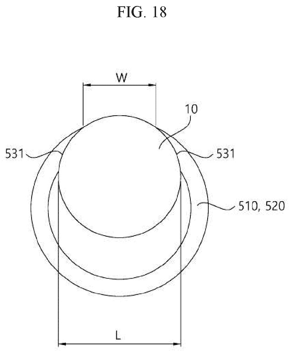

FIG. 16 is an enlarged perspective view of a locking portion according to one embodiment of the present disclosure, FIG. 17 is a view illustrating a clasp from various angles according to one embodiment of the present disclosure, and FIG. 18 is a cross-sectional view illustrating a state in which the clasp and a support wire are coupled according to one embodiment of the present disclosure. Hereinafter, a configuration of the locking portion according to one embodiment of the present disclosure will be described in greater detail, but not limited thereto, with reference to FIGS. 16 to 18.

As illustrated in FIG. 16, the mechanical thrombectomy device according to one aspect of the present disclosure may include the support wire 10, the clot arrestor 20, and the locking portion 30. In one aspect, the support wire 10 may be formed to have a structure as illustrated in FIG. 3 or a structure as illustrated in FIG. 16, but is not limited thereto. The support wire 10 may be tapered continuously or stepwise from a proximal portion toward a distal end to have a stiffness profile decreasing in the distal direction. In addition, the support wire 10 according to one embodiment of the present disclosure may include the stepped portion 200 at the distal end. In one aspect, the shape of the stepped portion 200 is not limited to those illustrated in FIGS. 3 and 16, and the stepped portion 200 may be, for example, a cylindrical shape not having the flat cross-section 210, or may be formed in any non-cylindrical shape or an irregular polyhedral shape.

In one aspect, as illustrated in FIGS. 3 and 16, the stepped portion 200 may have a maximum cross-sectional length greater than a maximum cross-sectional length of the support wire 10, and a step may be formed by an area difference caused by such a difference in maximum cross-sectional length between the support wire 10 and the stepped portion 200. The stepped portion 200 has a maximum cross-sectional length greater than that of the support wire 10, but may be tapered continuously or stepwise from a proximal portion toward a distal portion, similarly to the support wire 10. In one aspect, the stepped portion 200 may be tapered, for example, in a direction toward the proximal portion of the support wire 10 as illustrated in FIG. 16. In one aspect, edges of a surface of the stepped portion 200 may be ground or tapered so as to have an atraumatic surface that does not damage a blood vessel wall when the mechanical thrombectomy device is advanced, retrieved, or rotated in an operating region, thereby improving safety. In addition, grinding or tapering of the stepped portion 200 may reduce an abrupt change in the stepped portion 200 to form a smooth transition portion, thereby preventing an abrupt change in the radial profile of the locking portion 30.

The clot arrestor 20 according to one embodiment of the present disclosure includes the expandable frame 100 as illustrated in FIG. 1, and may be coupled to the support wire 10 by the locking portion 30. In one aspect, the clot arrestor 20 may have, at a proximal end thereof, a tail shape for coupling with the locking portion 30.

Referring to FIGS. 16 and 17, the locking portion 30 according to one embodiment of the present disclosure may include a clasp 500 configured to couple the support wire 10 and the clot arrestor 20. In one aspect, the clasp 500 may have a body with a semi-cylindrical shaped portion 501, and may include a first ring-shaped portion 510 at a first end of the semi-cylindrical shaped portion 501 and a second ring-shaped portion 520 at a second end of the semi-cylindrical shaped portion 501. As illustrated in FIG. 17, slits 530 may be formed in the first ring-shaped portion 510 and the second ring-shaped portion 520, respectively. Referring to FIG. 16, in one aspect, the clasp 500 at least partially surrounds a proximal portion of the clot arrestor 20 and a distal portion of the support wire 10, and the support wire 10 may be disposed in the respective slits 530 of the first ring-shaped portion 510 and the second ring-shaped portion 520. The clasp 500 may be formed of, for example, an alloy including cobalt and chromium, a super elastic nickel titanium alloy including nitinol, stainless steel, or the like.

FIG. 18 is a cross-sectional view illustrating a state in which the support wire 10 is inserted in the slit 530 of the first ring-shaped portion 510 or the second ring-shaped portion 520 of the clasp 500. In one aspect, each slit 530 of the first ring-shaped portion 510 and the second ring-shaped portion 520 of the clasp 500 according to one embodiment of the present disclosure may have opposing inclined surfaces 531 that contact a surface of the support wire 10. In one aspect, the inclined surfaces 531 may have a form of flat surfaces angled with respect to each other or a form of curved surfaces angled with respect to each other. Due to the inclined surfaces 531, the slit 530 may have different widths at a portion contacting the support wire 10 on an outer surface of the first or second ring-shaped portion 510 or 520 and at a portion contacting the support wire 10 on an inner surface of the first or second ring-shaped portion 510 or 520. According to one aspect of the present disclosure, a minimum width W of the slit 530 may be formed to be smaller than a maximum length L of a cross-section of the support wire 10. Since the minimum width W of the slit 530 is smaller than the maximum length L of the cross-section of the support wire 10, when the support wire 10 and the locking portion 30 are coupled, the support wire 10 may be prevented from being disengaged from the clasp 500 through the slit 530.

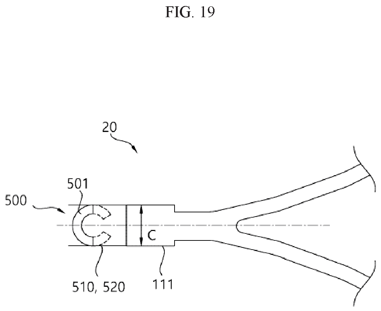

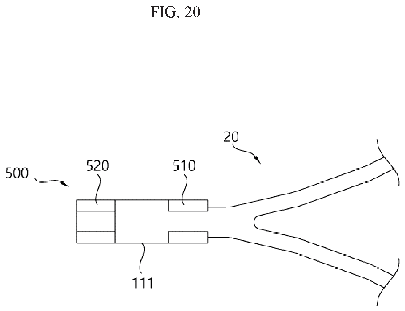

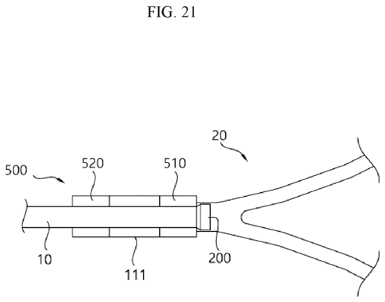

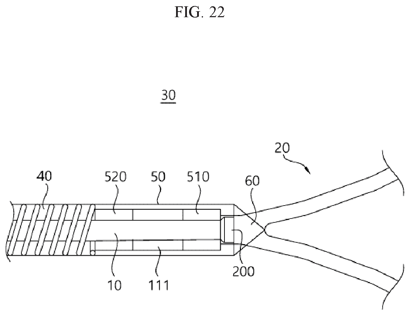

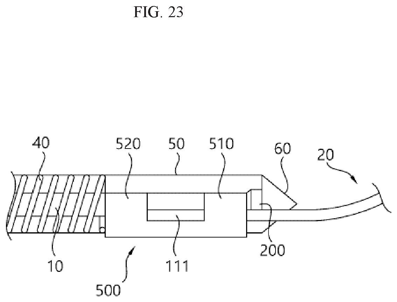

FIG. 19 is a view for explaining a proximal portion of a clot arrestor according to one embodiment of the present disclosure, FIG. 20 is a coupling view of the proximal portion of the clot arrestor and a clasp according to one embodiment of the present disclosure, FIG. 21 is a coupling view of the proximal portion of the clot arrestor, the clasp, and a support wire according to one embodiment of the present disclosure, FIG. 22 is a plan view of a locking portion according to one embodiment of the present disclosure, and FIG. 23 is a side view of the locking portion according to one embodiment of the present disclosure.

First, the proximal portion of the clot arrestor 20 according to one embodiment of the present disclosure will be described with reference to FIG. 19. As illustrated in FIG. 19, the clot arrestor 20 according to one embodiment of the present disclosure may have a tail shape having a structure in which the proximal portion may be caught on the locking portion 30 when coupled to the locking portion 30. For example, in one aspect, the proximal portion of the clot arrestor 20 may be coupled so as to be caught on the semi-cylindrical shaped portion 501 between the first ring-shaped portion 510 and the second ring-shaped portion 520 of the clasp 500. More specifically, the proximal portion of the clot arrestor 20 may include a T-shaped tail portion 111 fitted between the first ring-shaped portion 510 and the second ring-shaped portion 520 of the clasp 500.

According to one aspect of the present disclosure, a transverse length C of the T-shaped tail portion 111 perpendicular to the longitudinal direction (illustrated by a dashed-dotted line in FIG. 19) of the proximal portion of the clot arrestor 20 may be greater than inner diameters of the semi-cylindrical shaped portion 501, the first ring-shaped portion 510, and the second ring-shaped portion 520 of the clasp 500, and may be less than or equal to outer diameters of the semi-cylindrical shaped portion 501, the first ring-shaped portion 510, and the second ring-shaped portion 520 of the clasp 500, as illustrated in FIG. 19. More specifically, the T-shaped tail portion 111 according to one embodiment of the present disclosure may be placed so as to be fitted on a portion of the clasp 500 corresponding to a thickness of the semi-cylindrical shaped portion 501 between the first ring-shaped portion 510 and the second ring-shaped portion 520 of the clasp 500, as illustrated in FIG. 16. Such fitting of the T-shaped tail portion 111 is enabled because the transverse length C of the T-shaped tail portion 111 is greater than the inner diameter of the semi-cylindrical shaped portion 501. In addition, the transverse length C of the T-shaped tail portion 111 may be smaller than or equal to the outer diameter of the semi-cylindrical shaped portion 501. As a result, the T-shaped tail portion 111 is placed on the semi-cylindrical shaped portion 501 but does not protrude outward from the semi-cylindrical shaped portion 501, so that the radial profile of the locking portion 30 may not increase. In this regard, in one aspect, a distance between the first ring-shaped portion 510 and the second ring-shaped portion 520 of the clasp 500 may have a spacing corresponding to a longitudinal length of the T-shaped tail portion 111 so that the T-shaped tail portion 111 can be fitted therein.

Hereinafter, coupling of the support wire 10, the clot arrestor 20, and the locking portion 30 according to one embodiment of the present disclosure will be described in greater detail, but not limited thereto, with reference to FIGS. 20 to 23.

In the mechanical thrombectomy device according to one embodiment of the present disclosure, in order to couple the support wire 10, the clot arrestor 20, and the locking portion 30, first, the proximal portion of the clot arrestor 20 may be disposed in the clasp 500 as illustrated in FIG. 20. In this case, the T-shaped tail portion 111 of the proximal portion of the clot arrestor 20 may be disposed to be fitted on the semi-cylindrical shaped portion 501 between the first ring-shaped portion 510 and the second ring-shaped portion 520 of the clasp 500 as described above. Thereafter, in one aspect, the support wire 10 may be disposed on the proximal portion of the clot arrestor 20 as illustrated in FIG. 21. More specifically, the support wire 10 may be disposed in the slits 530 of the clasp 500 while covering the proximal portion of the clot arrestor 20 disposed to be fitted between the first ring-shaped portion 510 and the second ring-shaped portion 520, for example. In one aspect, the stepped portion 200 of the support wire 10 may be disposed to be caught on an outer surface of the first ring-shaped portion 510 of the clasp 500, and a distal portion of the support wire 10 excluding the stepped portion 200 may pass through the respective slits 530 of the clasp 500 and be disposed on at least a portion of the proximal portion of the clot arrestor 20 and the T-shaped tail portion 111. According to one aspect of the present disclosure, after the T-shaped tail portion 111 and the support wire 10 are disposed in the clasp 500 as described above, the clasp 500 may be tightened so that the first ring-shaped portion 510 and the second ring-shaped portion 520 contact a circumferential surface of the support wire 10 in order to fix the support wire 10 and the clot arrestor 20. By arranging and tightening the clasp 500 as described above, axial movement of the support wire 10 and the clot arrestor 20 can be prevented, and the support wire 10 and the clot arrestor 20 can be more securely coupled without being separated from the clasp 500.

Referring to FIG. 22, the locking portion 30 according to one embodiment of the present disclosure may further include a coil 40 wound around the support wire 10 to support the locking portion 30. In one aspect, the coil 40 may be disposed on the support wire 10 so as to be caught on an outer surface of the second ring-shaped portion 520 of the clasp 500, and may be firmly wound around the support wire 10, for example, with a diameter greater than the maximum cross-sectional length of the support wire 10 and smaller than or equal to the outer diameter of the clasp 500. In one aspect, the coil 40 may be disposed to be at least partially wound around the second ring-shaped portion 520 of the clasp 500, and the first ring-shaped portion 510 and the second ring-shaped portion 520 of the clasp 500 may be tapered or ground into a shape capable of closely contacting the coil 40 or the stepped portion 200 in order to prevent an abrupt increase in the radial profile of the locking portion 30. In one aspect, the coil 40 may be formed of stainless steel, platinum-iridium, or another radiopaque metal or material visible during fluoroscopy, and may act as a radiopaque marker.

Referring to FIGS. 22 and 23, in one aspect of the present disclosure, the clasp 500 may be disposed such that a first end thereof contacts the stepped portion 200 of the support wire 10 and a second end thereof contacts the coil 40, so as to be fixed. For example, the first end of the clasp 500 may be disposed to contact the tapered surface of the stepped portion 200, as illustrated in the plan view of FIG. 22. More specifically, referring to FIG. 23, the clasp 500 according to one embodiment of the present disclosure may be disposed such that the first end of the first ring-shaped portion 510 contacts the tapered surface of the stepped portion 200 so as to be fixed, in a state where the T-shaped tail portion 111 of the clot arrestor 20 is disposed to be fitted on the semi-cylindrical shaped portion 501 and the support wire 10 is disposed between the slits 530 while covering the T-shaped tail portion 111. In addition, as illustrated in FIG. 23, the second end of the clasp 500 according to one embodiment of the present disclosure may be disposed to at least partially contact the coil 40 so as to be fixed. As described above, in one aspect, the clasp 500 may act as the locking member that includes the T-shaped tail portion 111 of the clot arrestor 20 and the support wire 10 therein and couples them to each other. Accordingly, as illustrated in the side view of FIG. 23, the clasp 500 may be disposed to surround the distal portion of the support wire 10 and the proximal portion of the clot arrestor 20, and the first end and the second end are respectively fixed in contact with the stepped portion 200 and the coil 40, so that axial movement of the clasp 500 can be restricted. As a result, axial movement of the support wire 10 and the clot arrestor 20 coupled by the clasp 500 may also be prevented.

According to one aspect of the present disclosure, the locking portion 30 may further include soldering 50 applied to at least a portion of the coil 40, the distal portion of the support wire 10, the proximal portion of the clot arrestor 20, and at least a portion of the clasp 500, in order to further prevent separation of the distal portion of the support wire 10, the proximal portion of the clot arrestor 20, the coil 40, and the clasp 500. The soldering 50 may generally be formed of an alloy material including lead, tin, or silver, and in one aspect may be formed of a material including a radiopaque material to act as a radiopaque marker. For example, the soldering 50 may flow into gaps of at least a portion of the coil 40 wound around the support wire 10, and may be applied to surround the distal portion of the support wire 10, the proximal portion of the clot arrestor 20, and at least a portion of the clasp 500, thereby further strengthening coupling therebetween. In addition, a thickness, diameter, width, and length of the soldering 50 may be determined according to coupling strength and flexibility characteristics required for the mechanical thrombectomy device 1.

Referring to FIGS. 22 and 23, in one aspect, the soldering 50 applied to the distal portion of the support wire 10 and the proximal portion of the clot arrestor 20 may be wrapped by a biocompatible adhesive 60 at the stepped portion 200 of the support wire 10. The adhesive 60 may allow the stepped portion 200 of the support wire 10 and the proximal portion of the clot arrestor 20 to be more firmly attached to each other, and may smoothly finish an end of the soldering 50. According to one aspect, the biocompatible adhesive 60 may be tapered in a direction toward the clot arrestor 20. In one aspect, the adhesive 60 may be tapered to sufficiently cover the end of the soldering 50 and form a smooth curve, and such tapering may smooth the stepped portion 200 of the support wire 10, thereby reducing frictional force when the mechanical thrombectomy device 1 moves within a blood vessel and providing an atraumatic surface capable of preventing damage to a blood vessel wall.

Due to the various configurations of the locking portion and the locking mechanism of the present disclosure described above, the coupling between the distal end of the support wire 10 and the proximal end of the clot arrestor 20 can be reinforced, thereby providing the mechanical thrombectomy device 1 having high coupling strength. In addition, the thrombectomy device 1 may be provided in which the coupling strength of the locking portion 30 between the support wire 10 and the clot arrestor 20 is sufficiently high even during repeated deformation while the radial profile of the locking portion 30 that couples the support wire 10 and the clot arrestor 20 is maintained compact.

Although the present disclosure has been described above with reference to the drawings and embodiments, this does not mean that the scope of protection is limited by the drawings or embodiments. A person skilled in the art will understand that the present disclosure may be variously modified and changed without departing from the spirit and scope of the present disclosure described in the following claims.

The present disclosure described above is not limited by the above-described embodiments and the accompanying drawings, and it will be apparent to a person having ordinary skill in the art to which the present disclosure pertains that various substitutions, modifications, and changes may be made without departing from the technical spirit of the present disclosure.

Combinations of the above-described embodiments are not limited to the above-described embodiments, and various forms of combinations may be provided according to implementation and/or need, in addition to the above-described embodiments.

The above-described embodiments include examples of various aspects. Although all possible combinations for representing the various aspects cannot be described, a person having ordinary skill in the art will recognize that other combinations are possible. Accordingly, the present disclosure should be understood as including all other substitutions, modifications, and changes falling within the scope of the following claims.

**DESCRIPTION OF REFERENCE NUMERALS**

| | | | |
|---|---|---|---|
| 1: | mechanical thrombectomy device | 10: | support wire |
| 20: | clot arrestor | 30: | locking portion |
| 40: | coil | 50: | soldering |
| 60: | adhesive | 100: | expandable frame |
| 110: | proximal frame end | 111: | T-shaped tail portion |
| 200: | stepped portion | 210: | flat cross-section |
| 211: | plurality of first through-holes | 220: | catching surface |
| 300: | tail portion | 310: | cut-out |
| 311: | dual fins | 320: | branch |
| 321: | end | 322: | gap |
| 330: | ring-shaped portion | 331: | opening |
| 340: | second through-hole | 400: | locking mechanism |
| 410: | coil | 420: | soldering |
| 500: | clasp | 501: | semi-cylindrical shaped portion |
| 510: | first ring-shaped portion | 520: | second ring-shaped portion |
| 530: | slit | 531: | inclined surface |

## Claims

1. A mechanical thrombectomy device, comprising:
a support wire;
a clot arrestor including an expandable frame and connected to the support wire; and
a locking portion configured to couple the support wire and the clot arrestor,
wherein the locking portion includes a clasp having a first ring-shaped portion at a first end of a semi-cylindrical shaped portion and a second ring-shaped portion at a second end of the semi-cylindrical shaped portion, wherein a slit is formed in each of the first ring-shaped portion and the second ring-shaped portion.

2. The mechanical thrombectomy device of claim 1, wherein the clasp at least partially surrounds a proximal portion of the clot arrestor and a distal portion of the support wire, and
the support wire is disposed in the slit of each of the first ring-shaped portion and the second ring-shaped portion.

3. The mechanical thrombectomy device of claim 2, wherein the slit has opposing inclined surfaces that are in contact with a surface of the support wire.

4. The mechanical thrombectomy device of claim 3, wherein a minimum width of the slit is formed to be smaller than a maximum cross-sectional length of the support wire.

5. The mechanical thrombectomy device of claim 2, wherein the proximal portion of the clot arrestor is coupled so as to be engaged between the first ring-shaped portion and the second ring-shaped portion.

6. The mechanical thrombectomy device of claim 2, wherein the proximal portion of the clot arrestor includes a T-shaped tail portion fitted between the first ring-shaped portion and the second ring-shaped portion.

7. The mechanical thrombectomy device of claim 6, wherein a transverse length of the T-shaped tail portion perpendicular to a longitudinal direction of the proximal portion of the clot arrestor is greater than inner diameters of the semi-cylindrical shaped portion, the first ring-shaped portion, and the second ring-shaped portion of the clasp, and is smaller than or equal to outer diameters of the semi-cylindrical shaped portion, the first ring-shaped portion, and the second ring-shaped portion of the clasp.

8. The mechanical thrombectomy device of claim 5, wherein a distal end of the support wire includes a stepped portion having a maximum cross-sectional length greater than a maximum cross-sectional length of the support wire.

9. The mechanical thrombectomy device of claim 8, wherein the stepped portion is tapered in a direction toward a proximal portion of the support wire.

10. The mechanical thrombectomy device of claim 8, wherein the support wire is disposed in the slit while covering the proximal portion of the clot arrestor disposed between the first ring-shaped portion and the second ring-shaped portion.

11. The mechanical thrombectomy device of claim 8, wherein the locking portion further includes a coil wound around the support wire to support the locking portion.

12. The mechanical thrombectomy device of claim 11, wherein the clasp is disposed such that the first end contacts the stepped portion of the support wire and the second end contacts the coil, so as to be fixed.

13. The mechanical thrombectomy device of claim 12, wherein the locking portion further includes soldering applied to at least a portion of the coil, the distal portion of the support wire, the proximal portion of the clot arrestor, and at least a portion of the clasp so as to prevent separation of the distal portion of the support wire, the proximal portion of the clot arrestor, the coil, and the clasp.

14. The mechanical thrombectomy device of claim 13, wherein the soldering applied to the distal portion of the support wire and the proximal portion of the clot arrestor is wrapped by a biocompatible adhesive at the stepped portion of the support wire.

15. The mechanical thrombectomy device of claim 14, wherein the adhesive is tapered in a direction toward the clot arrestor.
